# EUROPEAN PATENT SPECIFICATION

(11) **EP 0 723 455 B1**
(45) Date of publication and mention of the grant of the patent: **09.04.2003**
(21) Application number: 94929970.5
(22) Date of filing: 30.09.1994
(51) Int. Cl.: A61K 38/00, A61K 31/70, A61K 48/00, A61P 37/00

(54) **METHOD OF INHIBITING PHAGOCYTOSIS**
VERFAHREN ZUR HEMMUNG DER PHAGOZYTOSE
METHODE D'INHIBITION DE LA PHAGOCYTOSE

(30) Priority: 30.09.1993 US 129381
(43) Date of publication of application: 31.07.1996
(73) Proprietor: THE TRUSTEES OF THE UNIVERSITY OF PENNSYLVANIA, Philadelphia, Pennsylvania 19104-3147 (US)
(72) Inventor: SCHREIBER, Alan D., Philadelphia, PA 19119 (US); PARK, Jong-Gu, Drexel Hill, PA 19026 (US)
(74) Representative: Crisp, David Norman
(86) International application number: US9411108
(87) International publication number: WO95009002

(56) References cited:
- EP-A- 0 614 978
- WO-A-91/06570
- WO-A-93/21317
- US-A- 4 686 282
- US-A- 5 087 617
- US-A- 5 189 014
- SAUT S C ET AL.: "Soluble FcgR (sFcgR): detection in biological fluids and production of a murine recombinant sFcgR biologically active ein vitro and in vivo" IMMUNOBIOLOGY, vol. 185, 1992, pages 207-221, XP002107201
- THE JOURNAL OF BIOLOGICAL CHEMISTRY, Volume 268, No. 21, issued 25 July 1993, A. AGARWAL et al., "Involvement of p72syk, A Protein-Tyrosine Kinase, in Fc gamma Receptor Signaling", pages 15900-15905.
- TIBTECH, Volume 8, issued July 1990, J. ROSSI et al., "RNA Enzymes (Ribozymes) as Antiviral Therapeutic Agents", pages 179-183.
- BLOOD, Volume 77, No. 3, issued 01 February 1991, W. AKERLEY III et al., "Neutrophil Activation Through High-Affinity Fc gamma Receptor Using a Monomeric Antibody with Unique Properties", pages 607-615.
- ONCOGENE, Volume 1, issued 1987, O. SHOHAT et al., "Inhibition of Cell Growth Mediated by Plasmids Encoding p53 Anti-Sense", pages 277-283.
- JOURNAL OF IMMUNOLOGY, Volume 150, No. 8, Part II, 1754, issued 15 April 1993, Z. INDIK et al., "Examination of Phagocytosis by Chimeric Fc gamma Receptors", page 306A.

## Description

### TECHNICAL FIELD

The present invention relates, in general, to pharmaceutical compositions usable for treating diseases resulting from interactions between immune complexes and Fc receptors. In particular, it can be used for modulating the clearance of antibody-coated cells, viruses, or soluble antigens by inhibiting phagocytosis, and to methods of modulating the interaction of immune complexes with cellular or tissue Fc receptors. The invention also relates to the modulation of those immune reactions for which the reaction of antigen-antibody complexes with Fc receptors is an important initiating step.

### BACKGROUND OF THE INVENTION

Certain immunological disorders are characterized by a disturbance in the expression of monocyte or macrophage Fc (IgG) receptors. An increase in the number of Fc receptors can result from an increase in the level of Fc receptor mediators such as gamma interferon or infection or the release of bacterial products. A decrease in the number of Fc receptors that can bind IgG can result not only from a reduction in the actual number of functional receptors but also from the saturation of Fc receptors by immune complexes. In certain autoimmune diseases, such as systemic lupus erythematosus, levels of circulating immune complexes can be high and thus receptor saturation can occur.

In autoimmune diseases, the body's mechanisms for distinguishing between itself and foreign invaders malfunction. Typically, the body begins to make antibodies to certain parts of itself; these antibodies trigger the immune system which then destroys the tissue identified by the abnormal antibodies.

Autoimmune diseases have varied focal points of attack. The autoimmune hemolytic anemias represent a group of disorders in which individuals produce antibodies to one or more of their own erythrocyte membrane antigens. Coating of erythrocytes by the abnormal antibodies is followed by their clearance from the circulation by splenic macrophages and subsequent destruction in the spleen. Representative diseases in this class are immune hemolytic anemia, immune thrombocytopenic purpura and autoimmune neutropenia. Another type of autoimmune disease is the type represented by systemic lupus erythematosus and rheumatoid arthritis. In these diseases, chronic inflammation is present in the joints, tendons, kidneys, lung, heart and other organs. In rheumatoid arthritis, for example, breakdown of joint cartilage into the synovial fluid of the joint is present in later stages of the disease. In systemic lupus erythematosus, however, cartilage or bone degradation is not usually found. Systemic lupus erythematosus and rheumatoid arthritis are often present in conjunction with other types of autoimmune disease. In systemic lupus erythematosus and rheumatoid arthritis, tissue destruction is associated with the presence of IgG-containing complexes in the circulation. It is believed that recognition of these complexes in tissues by cells having Fc receptors initiates or increases tissue destruction by macrophages and possibly other cells such as polymorphonuclear leukocytes in these tissues. Reaction with these Fc receptors initiates a range of immune-associated reactions that may harm body tissues in proximity to these Fc receptor bearing cells.

Diseases that involve the interaction of IgG-containing immune complexes with macrophage Fc receptors are often treated with corticosteroids, or immunosuppressants. These treatments can have diverse and serious side effects. The present invention offers alternative treatment approaches that can be used alone or in combination with more conventional drug therapies.

### SUMMARY OF THE INVENTION

It is a general aim of the invention to provide a pharmaceutical composition for modulating the clearance of antibody-coated cells or immune complexes, for example, by inhibiting the phagocytic potential of cells bearing Fc receptors.

It is a specific aim of the invention to provide compositions for regulating the clearance of immune complexes from a mammal. In addition, it is a specific object of the invention to provide a method of inhibiting the binding of immune complexes to membrane-bound Fc receptors (and/or inhibiting ingestion of such complexes), thereby inhibiting the sequelae of undesirable tissue damage.

It is a further aim of the invention to provide constructs and compounds suitable for use in the above-described methods.

According to one aspect of the present invention there is provided an *ex vivo* method of inhibiting the signal transduction of the γ subunit of the mast cell IgE receptor FcεRI comprising introducing into mast cells bearing said receptor an inhibitor of a kinase, which kinase is endogenous to said cells and activates said signal transduction of said FcεRI receptor, or the γ subunit thereof, by phosphorylation of the cytoplasmic domain of said γ subunit, said inhibitor being a peptide comprising the sequence Y-X2-L, wherein X2 represents any two amino acids, or a mimetic thereof and said introduction being effected under conditions such that said signal transduction is inhibited.

According to another aspect of the present invention there is provided a pharmaceutical composition, in the form of an aerosol, for inhibiting the signal transduction of the γ subunit of the mast cell IgE receptor FcεRI, comprising a carrier and an inhibitor of a kinase, which kinase is endogenous to said cells and activates said signal transduction of said FcεRI receptor, or the γ subunit therefor, by phosphorylation of the cytoplasmic domain of said γ subunit, wherein the inhibitor is present in the aerosol as a particle.

According to another aspect of the present invention there is provided use of an inhibitor of a kinase, which kinase is endogenous to mast cells bearing an IgE receptor FcεRI and activates signal transduction of said FcεRI receptor, or the γ subunit thereof, by phosphorylation of the cytoplasmic domain of said γ subunit, for the preparation of a medicament for treatment of an immunological disease or an immune mediated disease, wherein said inhibitor is a peptide comprising the sequence Y-X2-L, wherein X2 represents any two amino acids, or a mimetic thereof.

According to a further aspect of the present invention there is provided an inhibitor of a kinase, which kinase is endogenous to mast cells and phosphorylates the cytoplasmic domain of the γ subunit of the mast cell IgE receptor FcεRI, said inhibitor (i) being a peptide comprising a single tyrosine containing sequence required for signal transduction, said single tyrosine containing sequence being a Y-X2-L sequence, wherein X2 represents any two amino acids, or mimetic thereof, and (ii) inhibiting the signal transduction of said γ subunit when said inhibitor is introduced into said mast cells.

Further aims and advantages of the present invention will be clear from the description that follows. It will be appreciated that the disclosure should be read in light of the teachings available in the art relating to the isolation and cloning of the three classes of Fcγ receptors (FcγRI, FcγRII and Fcγ RIII) (see, for example, Allen and Seed, Science 243:378 (1989); Hibbs et al, Proc. Natl. Acad. Sci. USA 85:2240 (1988); J. Exp. Med. 166:1668 (1987); van de Winkle et al, FASEB J., 5:A964 (1991); Brooks et al, J. Exp. Med. 170:369 (1989); Stuart et al, EMBO J. 8:3657 (1989); Qui et al, Science 248:732 (1990); Simmons and Seed, Nature 333:568 (1988); see also, Schreiber et al, Clin. Immunol. Immunopath. 62:S66 (1992).

### BRIEF DESCRIPTION OF THE DRAWINGS

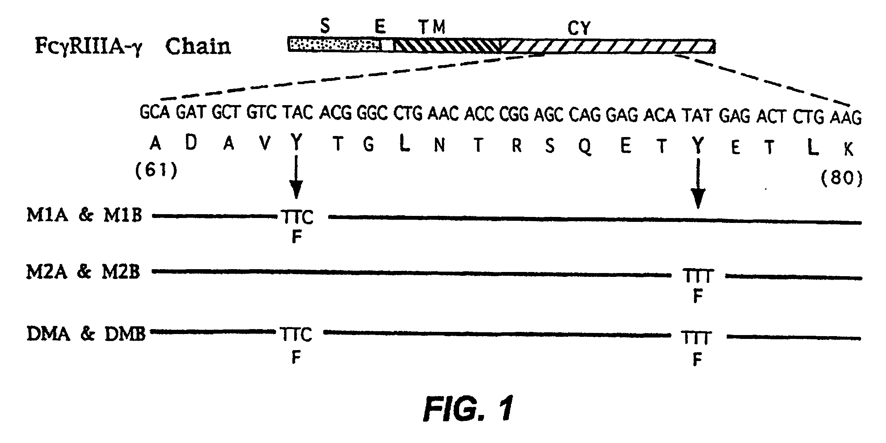

Figure 1 shows a schematic representation of FcγRIIIA γ wild type and mutants. Shown above the schematic diagram of the γ chain are signal sequence (S), external peptides (E), transmembrane domain (TM), and cytoplasmic domain (CY). The expanded area shows an area of the nucleotide sequence of the γ chain containing the conserved motif. In this Figure, the murine γ chain is shown. The conserved amino acids of the gene family of the γ and ζ chain genes are denoted by the underline. The N-proximal tyrosine encoded by the TAC codon of the nucleotides 235-237 (Ra et al, J. Biol. Chem. 264:15323 (1989)) was conservatively replaced with a phenylalanine encoded by TTC (clones M1A and M1B). Similarly, the C-proximal tyrosine encoded by TAT (168-270) was replaced with a phenylalanine encoded by TTT (clones M2A and M2B). For the double tyrosine-substitution mutants, both the N- and C-proximal tyrosines were replaced with phenylalanine (clones DMA and DMB). Solid lines of the mutants represent identical sequences to that of the wild type γ gene.

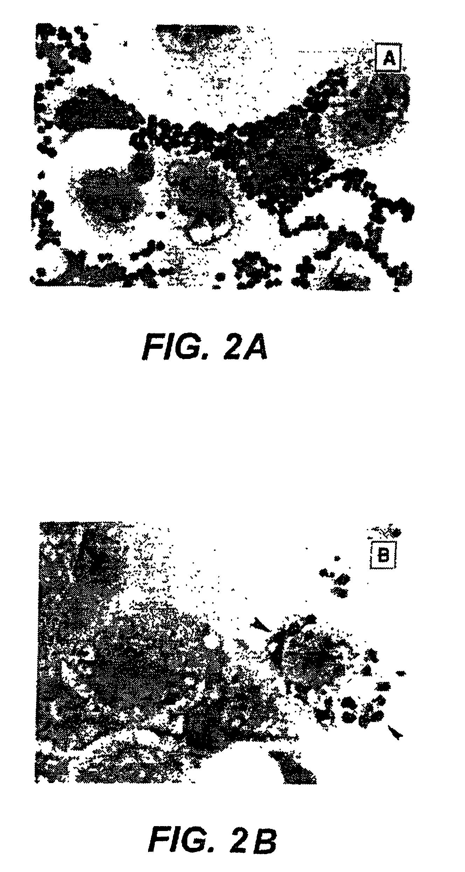

Figures 2A and 2B show binding and phagocytosis of IgG-sensitized RBCs (EA) by transfected COS-1 cells. Binding of EA by transfected COS-1 cells (left panel: A, C, E and G). Phagocytosis of EA by transfected COS-1 cells (right panel; B, D, F, and H). (A) and (B): binding and phagocytosis of COS-1 cells transfected with FcγRIIIA α and wild type γ. Three of the phagocytosed RBCs shown with wild type γ are marked by arrows in Figure (B), (C) and (D): transfectants containing α and γ (MIA). (E) and (F): transfectants containing α and γ (M2A). (G) and (H): transfectants containing α and γ (DMA). No phagocytosis of EA is seen in D, F and H. Pictures show images magnified by 1000x.

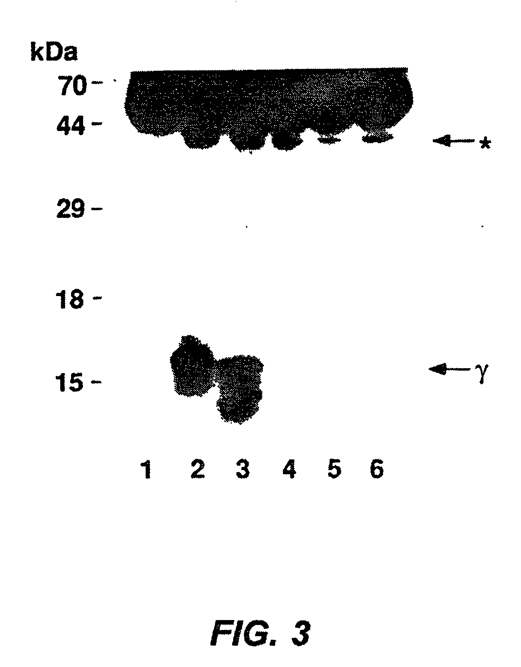

Figure 3 shows tyrosine phosphorylation of the wild type and mutant γ chains by *in vitro* kinase assay. The γ chain was immunoprecipitated with anti-γ antisera from lysates of COS-1 transfectants. *In vitro* phosphorylated samples were run on a 12.5% reducing SDS-PAGE gel. The gel was treated with IN KOH to remove phosphoserine and threonine, dried and the autoradiogram was examined after 4 days. lane 1: Sham transfectants with FcγRIIIA-α and pSVL vector without γ cDNA insert. lanes 2: FcγRIIIA α + wild type human γ. lane 3: FcγRIIIA α + wild type mouse γ. lane 4: FcγRIIIA α + M1A. lane 5: FcγRIIIA α + M2A. lane 6: FcγRIIIA α + DMA. The phosphorylated γ chains are denoted by an arrow (shown on the lower right side). The arrow with an asterisk (shown on the upper right side) is a specific tyrosine phosphoprotein band at approximately 40 kDa.

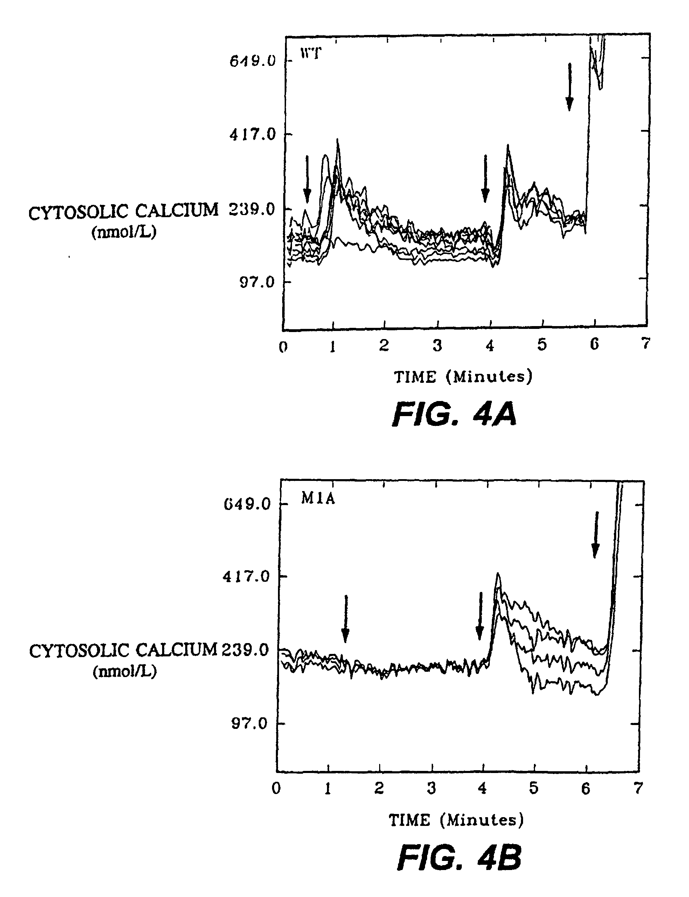

Figures 4A-4D are a Ca²⁺ mobilization following FcγRIIIA stimulation. Measurement of [Ca²⁺]i in individual cells was carried out during crosslinking of FcγRIIIA. The time points when anti-FcγRIII mAb, epinephrine (positive control) and calcium ionophore were added are denoted by arrows in each figure. Images were acquired at either 340 or 380 nm excitation (emission = 510 nm). 340/380 ratios were converted to [Ca²⁺]i based on calibration with Fura-2. The responses of M1A, M2A and DMA transfectants were greatly decreased compared to WT transfectants.

### DETAILED DESCRIPTION OF THE INVENTION

The present invention relates, at least in part, to pharmaceutical composition usable for modulating the clearance from a mammal (eg, from the circulation of a mammal) of antibody-coated cells. Accordingly, the invention provides methods of treating immunologic disorders, such as autoimmune diseases, characterized by interactions of immune complexes (eg, IgG-containing immune complexes) with Fc receptors (for example, those present on the surface of macrophage), and immune mediated diseases such as asthma. The methods of the invention result in Fc receptor expression and/or function being altered so that phagocytosis of IgG antibody-coated cells is reduced. (One skilled in the art will appreciate that patients suffering from immune complex diseases such as lupus erythematosus and rheumatoid arthritis may benefit from protocols designed so as to increase clearance of circulating immune complexes in the liver and spleen and thereby prevent their deposition in tissues such as the kidney and in the joints. This increase can be effected by stimulating liver and splenic macrophages using protocols for introducing sequences encoding Fc receptors described in the commonly owned application entitled "Methods of Stimulating Phagocytosis" filed concurrently herewith, the entire disclosure of which is incorporated herein by reference.)

More specifically, the invention provides methods of inhibiting mast cell IgE receptor FcεRI function by inhibiting phosphorylation of the cytoplasmic domain of the γ subunit of said receptor.

### Inhibition of Phosphorylation of Fc Receptors:

In one embodiment, the present invention describes a method of preventing ingestion (eg phagocytosis) of immune complexes (eg IgG-coated cells) by inhibiting phosphorylation of core sequences within the cytoplasmic domain of Fc receptors. Phosphorylation of cytoplasmic residues of FcγRIIA and the γ subunit of FcγRIIIA has been shown to be essential for signal transduction events involved in phagocytosis (Indik et al, Trans. Ass. Amer. Phys. 105:214 (1992); Park et al, Clin. Res. 41:324A (1993); Darby et al, Blood 79:352A (1992); Mitchell et al, Clin. Res. 41:189A (1993); Huang et al, J. Biol. Chem. 267:5467 (1992) ; Hunter et al, Clin. Res. 41:244A (1993); Park et al, J. Clin. Invest. in press (1993)). More specifically, phosphorylation of tyrosine residues present within the motif E-X8-D-X2-Y-X2-L-X12-Y-X2-L, present in the cytoplasmic domain of FcγRIIA, and the motif D/E-X2, 7-D/E-Y-X2-L-X7-Y-X2-L, present in the cytoplasmic domains of the γ and ζ chains of FcRIIIA, is required for phagocytic signal transduction (the numbers following the letter X denote the number of amino acids at that position; X can be any amino acid but X2 within a Y-X2-L is preferably the amino acids present in a Y-X2-L sequence of the cytoplasmic domain of FcγRIIA or the γ chain of FcγRIII). It appears that the second Y-X2-L of these core sequences (motifs) is particularly important for phagocytosis. The present invention contemplates the introduction into target cells of an inhibitor of the kinase(s) responsible for phosphorylation. In a specific embodiment, the inhibitor is a peptide that includes a sequence similar to, if not identical to, at least a functional portion of a tyrosine-containing motif (note, for example, the underlined portions of the motifs set forth above) and thus serves as a competitive inhibitor of the kinase(s). As an example, the inhibitor can take the form of an Fc receptor devoid of the extracellular domain or devoid of the extracellular and transmembrane domains. Alternatively, the inhibitor can be structurally distinct from the above motifs, or functional portions thereof, and can inhibit phosphorylation competitively or non-competitively (eg, a mimetic of the active peptide can be used having a structural conformation similar to the binding site of the active peptide). For mast cells, the sequences of the γ chain of FcεRI necessary for mediator release (eg, histamine, cytokines and leukotrienes) can be inhibited using this strategy.

The peptide inhibitor of the invention, or mimetic thereof, can be introduced into target cells directly, for example, using liposomes. (See also approaches described in Science 26:1877 (1993) for administration of peptides modified so as to render them capable of crossing cellular lipid membranes.) Alternatively, a DNA sequence encoding the peptide inhibitor can be introduced using gene therapy protocols so that the peptide is produced intracellularly.

The inhibitor or inhibitor encoding sequence can be administered to the cells of the lung, including macrophages, in the form of an aerosol. The inhibitor or inhibitor encoding sequence can be present in the aerosol as a particle (e.g. liposome, or non-infectious bacteria, for example, Listeria, in the case of the encoding sequence) that is phagocytosed by the pulmonary macrophages. Phagocytosis results in the introduction into the macrophages of the inhibitor or inhibitor encoding sequence. Viral vectors can also be used to introduce the peptide inhibitor encoding sequence of the invention into cells of the pulmonary tree. The vectors can be introduced as an aerosol and can take the form of a replication defective herpes or adenoviral vector. Retroviral vectors can also be used. (See, generally, Bajocchi et al, Nat. Genet. 3:229 (1993); Lemarchand et al, Circ. Res., 72:1132 (1993); Ram et al, Cancer Res. 53:83 (1993); Crystal, Am. J. Med. 92:445 (1992); Yoshimura et al, Nucl. Acids Res. 20:3233 (1992); Morecki et al, Cancer Immunol. Immunother. 32:342 (1991); Culver et al, Hum. Gene Ther. 1:399 (1990); Culver et al, Transplant. Proc., 23:170 (1991).)

Blood monocytes can be transformed (infected) *ex vivo* with the peptide inhibitor encoding sequence of the invention and then reintroduced into the patient so that the inhibitor is produced *in vivo.*

An alternative approach to inhibiting phosphorylation involves the use of ribozymes that recognize RNA sequences specifying Fc receptor phosphorylation sites (eg, in FcγRIIA and/or in the γ subunit of FcγRIIIA), as well as RNA sequences specifying enzyme active sites. Introduction of the ribozyme can be effected using a carrier such as a liposome coated with IgG so as to direct insertion to Fcγ receptor bearing cells. Alternatively, IgE-coated liposomes can be used to direct the ribozyme to mast cells or basophiles, or cells of that lineage, bearing the IgE receptor FcεRI with its associated γ subunit. One skilled in the art will appreciate that this is an approach suitable for use in treating allergic disorders. The γ subunit of the IgE receptor is responsible for transmitting the signal inducing the release of intracellular mediators by mast cells. The destruction of the γ chain RNA is predicted to inhibit the release of these bioactive products.

In accordance with the above approach, ribozymes administered as described would bind to a few selected sequences (eg, RNA splicing and 5' untranslated sequences for which they were specific, for example, in FcγRIIA RNA or FcγRIIIA γ chain RNA) and the enzymatic activity associated with the ribozyme would result in digestion and thus removal of the RNA specifying functional sequences of the receptor necessary for phagocytic signal transduction. For mast cells, RNA sequences specifying the sequences of the γ chain of FcεRI necessary for mediator release (eg, histamine, cytokines and leukotrienes) can be eliminated using this strategy.

Where advantageous, continuous *in vivo* production of the ribozyme can be effected using *ex vivo* constructed packaging cells (eg, Psi2-like cells; see Miller and Rosman, Biotechniques 7:980, 1989 and Current Protocols in Molecular Biology III:9.1, 1992 (Supp. 17)). One skilled in the art will appreciate that a suicide gene can be included in such a cell so that ribozyme production can be terminated.

A further approach to inhibiting receptor phosphorylation involves the use of an antisense construct or ribozyme that targets Syk encoding sequences. The Syk gene product, but not the gene product of ZAP-70 of the Syk kinase family, has been shown to stimulate FcγRI and FcγRIIIA phagocytosis mediated by both the γ and ξ chains. Thus, by targeting Syk sequences, inhibition of Syk dependent phosphorylation can be effected. Constructs and ribozymes suitable for use in this method can be readily selected by one skilled in the art (see Yagi et al, Biochem. Biophys. Res. Comm. 200:28 (1994) for Syk gene sequence).

The following non-limiting Examples describe certain aspects of the invention in greater detail.

### EXAMPLE I

### Production of Recombinant Soluble FcγRIII

Recombinant soluble FcγRIII proteins can be produced using expression vectors as described below. The soluble protein can correspond to FcγRIII with the transmembrane domain removed. The constructs can be introduced into mammalian cells under conditions such that expression of the receptor encoding sequence occurs. The recombinant proteins thus produced are isolated both from the cell lysates and from the supernatants.

### Transfection of adherent cells or cells in suspension:

Transfection of adherent cells, eg, CHO cells or COS cells, or an appropriate suspension cell system will be performed. Permanent transfectants expressing soluble forms of Fcγ receptor will be established by electroporation, calcium phosphate or other established methods. Transfected cells will be allowed to grow 48 hours and selected in media containing Geneticin at 2 mg/ml (Gibco BRL, Gaithersburg, Maryland) or other selection drug. After approximately twelve weeks, positive colonies will be isolated and expanded for further characterization of the clones. The isolated clones will be examined by enzyme-linked immunoassay (ELISA) using ELISA plates (Dynatech, Alexandria, Virginia) to select a transfectant cell line expression the highest quantity of the soluble receptor. Mass culture of adherent transfectants will be achieved by employing the hollow-fiber tissue culture system.

### EXAMPLE II

### Function of Soluble FcγRIII

The functions of soluble FcγRIII proteins are assessed both *in vitro* and *in vivo.* The effect of soluble Fc receptors on IgG-immune complex binding to cellular membrane-bound receptors depends on several factors including the local concentrations of the ligand and soluble receptor, the surface density of the membrane-bound receptor, the valence of the ligand and the relative affinities of the two receptor forms for ligand. The limiting factors in the interaction of soluble FcγRIII receptors with ligand and cellular membranes can be deciphered using available model systems.

The *in vitro* assay systems rely on the competition of soluble receptors with cell membrane receptors for labeled IgG ligand and IgG-coated erythrocytes (EA). Fcγ receptor-negative cells are transfected with transmembrane FcγRIII molecules that retain the functional capacity to bind and ingest IgG-containing immune complexes and antibody-coated cells (Ruiz and Schreiber, J. Clin. Invest. 88:149 (1991)). These assays are used to examine the function of soluble receptors and the ability of soluble receptors to interfere with membrane receptor detection of both EA and oligomeric forms of IgG. The function of soluble FcγRIII is also examined *in vivo.* In these studies, an established experimental animal model is used to study whether soluble FcγRIII administered *in vivo* alters the clearance of antibody coated cells (Ruiz and Schreiber, J. Clin. Invest. 88:149 (1991)). The immunoregulatory potential of soluble FcγRIII is examined in this manner.

### EXAMPLE III

### Cytoplasmic Tyrosine Residues Required For Phagocytic Signal Mediation

### Experimental Protocols:

### Plasmid construction and introduction of point mutations:

The pSVL eucaryotic expression vector (Pharmacia LKB, Piscataway, NJ) was employed for expression of FcγRIIIA in COS-1 cells. huFcγRIIIA α cDNA was cloned into the Xbal and BamHI cloning sites of pSVL. Similarly, muFcγRIIIA γ cDNA was cloned into XhoI and BamHI cloning sites. TCR/FcγRIIIA ζ was cloned into the Xbal and BamHI cloning sites of pSVL. Conservative replacement of cytoplasmic tyrosines of the γ chain by phenylalanine was achieved using the two step overlap-extension polymerase chain reaction (PCR) (Horton et al, Biotechniques 8:528 (1990)). Double tyrosine substitution mutants were constructed sequentially by the substitution of the N-terminal tyrosine residue followed by the substitution of the C-terminal tyrosine residue. Six clones from each mutant were isolated and subjected to DNA sequencing. Two clones from each tyrosine substitution were randomly selected for further studies from several clones with correct DNA sequence.

### Transient transfection:

FcγRIIIA isoforms, FcγRIIIA-γγ, FcγRIIIA-ζζ, were generated by cotransfection of COS-1 cells with cDNA of γ or ζ as well as cDNA of α. Transfections of cDNAs were carried out with a modified DEAE-Dextran method. Briefly, 300,000 COS-1 cells were seeded on 35 mm well plates 24 hours prior to transfection. Plates of 70 to 80 % confluence were washed twice and incubated for 30 minutes with Dulbeco's Modification of Eagle's Medium (DMEM, Gibco BRL, Grand Island, NY) before transfection. Four µg of plasmid DNA (0.5 µg/µl) was slowly added to 1 ml of a transfection buffer containing Nu medium (DMEM with 10 % of NuSerum [Collaborative Biomedical, Two Oak Park, Bedford, MA], 1 mg/ml of DEAE Dextran and 100 µM chloroquine. The transfection buffer containing DNA was added to COS-1 cells with incubation for 4 hours at 37°C. Cells were then shocked with 10% DMSO in phosphate buffered saline (PBS) for 2 minutes, washed twice with DMEM and grown in NuSerum supplemented DMEM. Cells were studied 48 hours following transfection.

### Immunofluorescence staining and flow

*cytofluorimetry*: Transfected cells were harvested with staining buffer (PBS containing 0.02 % sodium azide and 0.1% BSA) using transfer pipettes. Cells were centrifuged, resuspended in 60 µl of staining buffer and incubated with either the anti-FcγRIII mAb, 3G8 (Unkeless et al, Annu. Rev. Immunol. 6:251 (1988)), or an isotype control for 30 minutes at 4°C. Cells were washed and stained with fluorescein-conjugated goat anti-mouse IgG (Tago Inc. Burlingame, CA). The stained cells were examined using a FACStar flowcytometer (Becton Dickinson Co., Mountain View, CA).

### Binding and phagocytosis of IgG-sensitized RBCs

*(EA)*: Sterile sheep red blood cells (10⁹/ml) in calcium and magnesium-free PBS were sensitized by incubation with an equal volume of a subagglutinating titer of rabbit anti-sheep RBC antibody (Cappel Laboratories, Cochranville, PA). The IgG-sensitized RBCs (EA) were washed twice with PBS and resuspended to a final concentration of 10⁹/ml for overlaying on transfected COS-1 cells. Cells were examined for rosetting (〉 10 EA per COS-1 cell) and phagocytosis as described previously (Indik et al, J. Clin. Invest. 88:A66 (1991)). For the analysis of phagocytosis, COS-1 cells bound with EA (after three washings) were subjected to a brief hypotonic shock (35 seconds) with hypotonic PBS to remove surface bound EA. The cells were then stained with Wright-Giemsa staining solutions, and phagocytosis (ingested EA) was determined by light microscopy. Results obtained were analyzed by Student's T-test.

### In vitro kinase assay:

Transfected cells (2 X 10⁷ cells) were washed once with PBS and incubated sequentially on ice with 5 µg/ml each of anti-FcγRIII mAb and goat anti-mouse IgG for 10 minutes. Cells were washed once with PBS and incubated at room temperature for 3 minutes before adding 1.5 ml of lysis buffer (150 mM NaCl, 25 mM Hepes [pH 7.4] and 1% polyoxyethylene 10 oleyl ether [BRIJ-96; Sigma, St. Louis, MO]) containing phosphatase and protease inhibitors. Inhibitors of phosphatases and proteases (1mM EGTA, 1 mM Na orthovanadate, 1 mM PMSF, 10 µg/ml aprotinin, 50 µg/ml leupeptin, and 100 µg/ml soybean trypsin inhibitor) were added fresh to lysis buffer. After 15 minutes of lysis on ice, cell lysates were centrifuged for 30 minutes at 4°C to clarify. The FcγRIIIA-γ chain was immunoprecipitated with anti-human γ antiserum (provided by Jean-Pierre Kinet, NIAID-NIH, Rockville, MD) and Protein A-sepharose CL4B (Signa, St. Louis, MO) in lysis buffer. Pellets were washed three times in lysis buffer and once in low salt buffer (100 mM NaCl, 25 mM Hepes, pH 7.4 and 5 mM MnCl₂). Pellets were incubated (20°C, 10 min.) with 30 µl of a mixture containing 25 mM Hepes, pH 7.4, 5 mM MnCl_{2'} 5 mM p-nitrophenyl-phosphate, 1 µM cold ATP (Boehringer Mannheim, Indianapolis, IN) and 5 µCiγ-[³²P]ATP (6000 Ci or 222 TBq/mmol; Dupont NEN, Boston, MA). Reactions were stopped by adding reducing SDS-PAGE sample buffer and labelled proteins were separated on a 12.5% reducing SDS-PAGE gel. The gel was fixed in methanol/acetic acid, treated with 1 N KOH (2 hrs at 55°C) to remove phosphoserine and threonine, dried and autoradiogrammed for 4 days.

### [Ca²⁺]i Mobilization:

COS-1 cells plated on glass coverslips were incubated with 2 µM Fura-2/AM (Calbiochem. San Diego, CA) for 30 minutes, washed twice and the coverslips then transferred to a Leidem cell chamber (Medical Systems, Greenville, NY) for multiple single-cell measurements of [Ca²⁺]i. FcγRIIIA receptors were crosslinked either with biotinylated anti-FcγRIII followed by the addition of streptavidin or with anti-FcγRIII mAB 3G8 whole IgG. As a positive control, 10 µM epinephrine was added to crosslink epinephrine receptors expressed on COS cells. Calcium imaging was performed using a 40x fluorescence objective on a Nikon Diaphot microscope with the image-1 AT quantitative fluorescence system (Universal Imaging, West Chester, PA). Images were acquired at either 340 or 380 nm excitation (emission = 510 nm). 340/380 ratio images were calculated on a pixel by pixel basis and the average 340/380 ratio within each cell determined at each time point. 340/380 ratios were converted to [Ca²⁺]i based on solution calibration using free Fura-2 acid.

### Phagocytosis Mediated by FcγRIIIA α and Associated γ and ζ Chains:

Wild type γ and ζ cDNAs of FcγRIIIA were cotransfected with the FcγRIIIA-α chain into COS-1 cells to examine their ability to induce phagocytosis of EA (sensitized RBC). Surface expression of FcγRIIIA was determined by flow cytometry and was equally efficient in cotransfection with either γ or ζ (Table 1). The mean fluorescence intensity (FMI) for cotransfected cells stained with anti-FcγRIII mAB increased by 15 fold compared to cells stained with an IgG isotype control or compared to mock-transfected cells stained with anti-FcγRIII mAB (Table 1). The transfectants were examined for their ability to bind and phagocytose IgG sensitized RBCs (EA). Approximately 50% of COS-1 transfectants avidly bound EA (Table 1). Microscopic examination of COS-1 cells transfected with wild type γ consistently showed the ingestion of EA by 20±5 % of the cells examined (p≺0.02). Thus, phagocytosis of EA was detected in approximately 40% of COS-1 cell transfectants that bound EA. In contrast, cotransfectants containing the ζ chain revealed 3.8% of cells with ingested EA (p≺0.02) (Table 1). Moreover, in ζ-containing cells which demonstrated phagocytosis the average number of ingested EA per cell was reduced to less than one half the level of that observed with γ. COS-1 cells transfected with all three cDNAs, α, γ, and ζ, revealed 16% cells with ingested EA, showing consistent attenuation in phagocytosis (Table 1). In contrast, neither sham transfectants with EA nor transfectants with E (non-sensitized RBC) exhibited any binding or phagocytosis.

**TABLE 1.**

| *FcγRIIIA expression and Phagocytosis by COS-1 Cells* *Transfected with FcγRIIIA (γ and*/*or ζ)*. | | | | |
|---|---|---|---|---|
| FcγRIIIA | MFI* | PI^{§} | Phagocytosis (% Cells +) | Rosetting (% Cells +) |
| α + pSVL (Sham) | 15 | 0 | 0 | 0 |
| | | | | |
| α+γ | 254 | 129±21.0 | 20±5.0 | 48±3.0 |
| | | | | |
| α+ζ | 220 | 19±3.2 | 3.8±0.7 | 50±1.7 |
| | | | | |
| α+ζ+γ | 205 | 77±5.0 | 16±3.2 | 46±2.0 |

Transfection efficiency was determined by flow cytometry. The mean fluorescence intensity (MFI) is shown for one of 3 separate experiments with similar results. Internalized RBCs were microscopically scored (1000x). Results are expressed as the mean ± SEM for phagocytosis and binding (rosetting) of EA. At least 3 separate experiments were performed for each clone. For each experiment, 1500 cells were counted at 5 randomly selected sites. *Mean Fluorescence Intensity. ^{§}PI (Phagocytic Index): number of RBCs internalized per 100 COS-1 cells

### Two Cytoplasmic Tyrosines of the γ Chain are Required for Phagocytosis:

To study the effect of the two conserved γ chain tyrosines on FcγRIIIA mediated phagocytosis, the N-proximal (clones M1A and M1B) or C-proximal (clones M2A and M2B) tyrosines were individually replaced by phenylalanine. For mutants with double tyrosine substitutions, both tyrosines were replaced by phenylalanine (DMA and DMB) (Fig. 1).

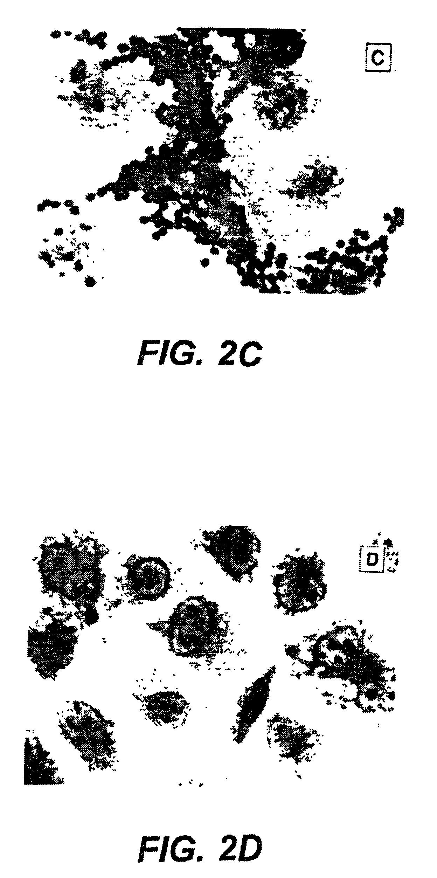

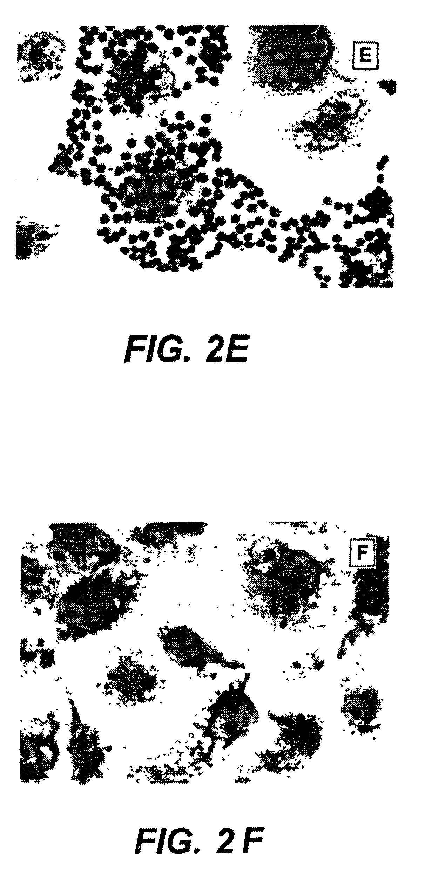

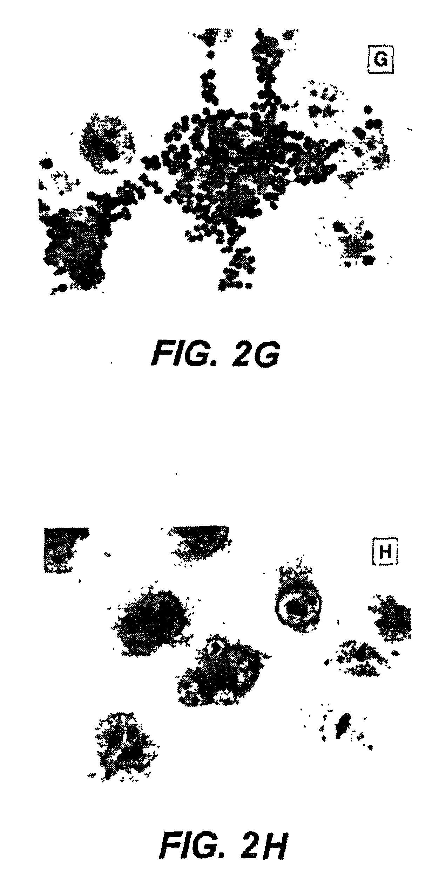

MFI measured by flow cytofluorimetry and % of positive cells with rosetting demonstrated similar surface expression of the receptor complexes in all transfectants bearing γ mutants and wild type γ (Table 2). These comparable levels of expression indicate that tyrosine residues in the cytoplasmic tail of the γ chain are not necessary for formation of the FcγRIIIA receptor complex required for surface expression. Results summarized in Table 2 are as follows: M1 γ mutants showed more than 99% reduction in phagocytic activity as shown by phagocytic index (PI) (≤ 1 % of transfectants with ingested EA and minimal ingested EA per phagocytosing cell)(p〈0.02); M2 and DM γ mutants demonstrated essentially no phagocytosis (1 among 5000 cells examined) (Table 2, Fig. 2).

**TABLE 2.**

| *FcγRIIIA expression and Phagocytosis by COS-1 Cells* *Transfected* with *FcγRIIIA -α*/*γ(wild type or mutants).* | | | | |
|---|---|---|---|---|
| FcγRIIIA | MFI* | PI^{§} | Phagocytosis (% Cells +) | Rosetting (% Cells +) |
| α+pSVL (Sham) | 15 | 0 | 0 | 0 |
| α+γ(WT) | 254 | 129±21.0 | 20±5.0 | 49±3.0 |
| α+γ(M1A) | 259 | 0.3±0.2 | 0.2±0.1 | 49±2.5 |
| α+γ(M1B) | 303 | 1.0±1.0 | 1.0±1.0 | 50±1.5 |
| α+γ(M2A) | 232 | ≤0.04 | ≤0.02 | 49±1.5 |
| α+γ(M2B) | 236 | ≤0.02 | ≤0.02 | 48±3.0 |
| α+γ(DMA) | 222 | ≤0.02 | ≤0.02 | 48±2.5 |
| α+γ(DMB) | 328 | ≤0.02 | ≤0.02 | 49±2.0 |
| See Table 1 for legend | | | | |

### Inhibition of Phagocytosis by Tyrophostin 23:

To investigate whether phagocytosis requires phosphorylation of tyrosine residues, COS-1 cells cotransfected with FcγRIIIA-α and wild type γ were incubated with increasing concentrations of tyrphostin 23 (tyr 23), an inhibitor of tyrosine kinases (Yaish et al, Science 242:933 (1988)). Tyr 23 decreased phagocytosis in a dose dependent manner, with 50% inhibition at 25 µM and complete inhibition at 200-400 µM (p≺0.01)(Table 3). In contrast, tyr 23 did not affect the binding of EA. Inhibition of phagocytosis was not associated with reduction in viability, since transfectants pretreated with tyr 23 (400 µM) followed by washing had phagocytic activity partially (3 hr wash, Table 3) or completely (overnight wash, data not shown) restored.

**TABLE 3.**

| *The Effect of Tyrphostin 23 (Tyr 23) on Phagocytosis by COS-1 Cells Transfected* with *FcγRIIIA-α*/*γ* | | |
|---|---|---|
| Tyr 23 (Concentration) | PI* | Rosetting (% Cells) |
| 0 µM | 125±24 | 49±3 |
| 25 µM | 68±4 | 52±9 |
| 50 µM | 26±7 | 52±8 |
| 100 µM | 16±6 | 49±7 |
| 200 µM | 1.2±1 | 47±5 |
| 400 µM | 0 | 48±3 |
| 400 µM + washing | 63±7 | 44±6 |

| | | |
|---|---|---|
| *PI. Phagocytic Index | | |

### Tyrosine Residues of the γ Subunit are Phosphorylated In Vitro:

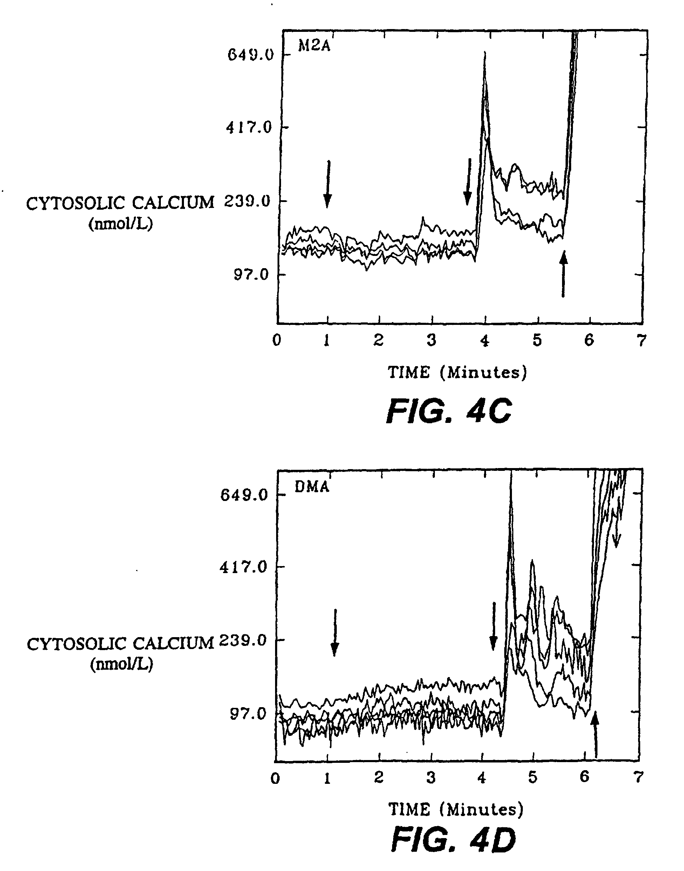

The possibility that tyrosine residues of the ζ chain are phosphorylated was examined by *in vitro* kinase assays using COS-1 transfectants. Results shown in Fig. 4 demonstrate that the tyrosine residues of the wild type γ chains are phosphorylated in vitro. In contrast, the mutant γ chain transfectants and the sham transfectants showed no detectable phosphorylation. Since the single tyrosine substitution mutants (M1A and M2A) did not exhibit phosphorylation on the remaining tyrosine residues, it is likely that phosphorylation of either one of the two tyrosine residues requires the other tyrosine residue to be intact (Fig. 3). These phosphorylation data correlate well with the ability of the γ chain to induce a phagocytic signal, as substitution of either one of the tyrosine residues largely eliminates phagocytosis (Table 2, Fig. 2).

The *in vitro* kinase assay demonstrated a distinct band of approximately 40 kDa present in all lanes except the sham transfectants. This band may represent an associated phosphoprotein coprecipitating with γ.

### Cytoplasmic Tyrosines of γ are Required for Mobilization of Ca²⁺:

To examine whether the γ chain tyrosines are required for calcium mobilization, the calcium response following FcγRIIIA crosslinking was measured in individual transfected cells (WT, M1A, M2A or DMA) using digital video microscopy (Fig. 4). Epinephrine, which evokes a Ca²⁺ signal in COS cells, was used as a positive control in all experiments. Transfectants with the WT receptor complex showed a typical transient calcium rise following cross-linking with biotinylated anti-FcγRIII followed by the addition of streptavidin or with anti-FcγRIII whole IgG. In 5 consecutive experiments (169 cells), 58% of cells responded to anti-FcγRIII with a calcium signal at least 50% as large as than induced by 10 µM epinephrine (Fig. 4, Table 4). In contrast, COS-1 cells transfected with either M1A, M2A or DMA showed markedly diminished calcium responses to anti-FcγRIII, although in one of four experiments significant calcium mobilization was evoked in M1A transfected COS-1 cells.

**TABLE 4.**

| *The Effect of Tyrosine Substitutions on Calcium Mobilization Evoked by Cross-Linking of FcγRIIIA* | | | |
|---|---|---|---|
| FcγRIII | No. of Experiments | No. of Cells | % of Cells Responding* |
| α+γ(WT) | 5 | 169 | 57.8 |
| | | | |
| α+γ(M1A) | 4 | 123 | 16.0 |
| | | | |
| α+γ(M2A) | 4 | 117 | 2.3 |
| | | | |
| α+γ(DMA) | 4 | 70 | 3.7 |

| | | | |
|---|---|---|---|
| * Cells were scored as responding if the calcium response was more than 50% of that observed with 10 µM epinephrine | | | |

### EXAMPLE IV

### Macrophage FcγRIII Signaling Induces Protein Tyrosine Kinase Activation

Specific tyrosine residues in the intracellular FcγRIIIγ subunit have been identified as necessary for signal transduction and subsequent effector functions, using NK cells and lymphocytes or fibroblasts transfected with chimeric or mutated receptors. (Darby et al, Blood 79:352A Nov. (1992)) FcγRIII in its native state on pulmonary macrophage or cultured monocytes (M) was examined in order to study the physiologically relevant protein tyrosine kinases (PTK) and phosphotyrosine containing substrates during macrophage signal transduction. Within seconds after FcγRIII crosslinking with Fab antibody, Western blot analysis revealed a characteristic pattern of phosphotyrosine substrates. This response was transient with most substrates peaking at 5 min. and declining after 10-20 min. Phosphotyrosine patterns were indistinguishable in fresh macrophage and cultured monocytes, validating the latter as a useful *in vitro* model. P62, a protein associated with p120^{ras}GAP, although not GAP itself, was identified by specific immunoprecipitation as one of these phosphotyrosine substrates. A second substrate was found to be p95^{vav}, a hematopoietic oncogene product which is also tyrosine phosphorylated after TCR, slg and FcεR1 activation. The kinase PTK72/Syk, heretofore identified only in B cell slg and mast cell FcεRI signaling, was also a major phosphotyrosine substrate after macrophage FcγRIII activation. *In vitro* kinase assays of anti-Syk immune complexes revealed a 3-4 fold increase in Syk autophosphorylation at 5-10 min. after receptor ligation. Syk has also been found to be present in immunoprecipitates of the γ chain FcγRIIIA suggesting that Syk is associated with phosphorylated γ chain.

While the invention has been described in connection with what is presently considered to be the most practical and preferred embodiment, it is to be understood that the invention is not to be limited to the disclosed embodiment, but on the contrary, is intended to cover various modifications and equivalent arrangements included within the scope of the appended claims.

## Claims

1. An *ex vivo* method of inhibiting the signal transduction of the γ subunit of the mast cell IgE receptor FcεRI comprising introducing into mast cells bearing said receptor an inhibitor of a kinase, which kinase is endogenous to said cells and activates said signal transduction of said FcεRI receptor, or the γ subunit thereof, by phosphorylation of the cytoplasmic domain of said γ subunit, said inhibitor being a peptide comprising the sequence Y-X2-L, wherein X2 represents any two amino acids, or a mimetic thereof and said introduction being effected under conditions such that said signal transduction is inhibited.

2. The method according to claim 1 wherein X2 represents the amino acids of a Y-X2-L sequence of the cytoplasmic domain of the γ chain FcεRI.

3. A pharmaceutical composition, in the form of an aerosol, for inhibiting the signal transduction of the γ subunit of the mast cell IgE receptor FcεRI, comprising a carrier and an inhibitor of a kinase, which kinase is endogenous to said cells and activates said signal transduction of said FcεRI receptor, or the γ subunit therefor, by phosphorylation of the cytoplasmic domain of said γ subunit, wherein the inhibitor is present in the aerosol as a particle.

4. The composition according to claim 3 wherein the particle is a liposome.

5. The composition according to claim 3 or claim 4 wherein said inhibitor is a peptide or mimetic.

6. The composition according to claim 5 wherein said peptide comprises the sequence Y-X2-L, wherein X2 represents any two amino acids.

7. The composition according to claim 6 wherein X2 represents the amino acids of a Y-X2-L sequence of the cytoplasmic domain of the γ chain FcεRI.

8. Use of an inhibitor of a kinase, which kinase is endogenous to mast cells bearing an IgE receptor FcεRI and activates signal transduction of said FcεRI receptor, or the γ subunit thereof, by phosphorylation of the cytoplasmic domain of said γ subunit, for the preparation of a medicament for treatment of an immunological disease or an immune mediated disease, wherein said inhibitor is a peptide comprising the sequence Y-X2-L, wherein X2 represents any two amino acids, or a mimetic thereof.

9. The method according to claim 8 wherein X2 represents the amino acids of a Y-X2-L sequence of the cytoplasmic domain of the γ chain FcεRI.

10. An inhibitor of a kinase, which kinase is endogenous to mast cells and phosphorylates the cytoplasmic domain of the γ subunit of the mast cell IgE receptor FcεRI, said inhibitor (i) being a peptide comprising a single tyrosine containing sequence required for signal transduction, said single tyrosine containing sequence being a Y-X2-L sequence, wherein X2 represents any two amino acids, or mimetic thereof, and (ii) inhibiting the signal transduction of said γ subunit when said inhibitor is introduced into said mast cells.

11. An inhibitor according to claim 10 wherein X2 represents the amino acids of a Y-X2-L sequence of the cytoplasmic domain of the γ chain FcεRI.

## Patentansprüche

1. *Ex Vivo*-Verfahren zum Hemmen der Signalweiterleitung der γ-Untereinheit des Mastzellen-IgE-Rezeptors FcεRI, bei dem man in Mastzellen, die den Rezeptor tragen, einen Inhibitor für eine Kinase einbringt, wobei die Kinase endogen für die Zellen ist und die Signalweiterleitung des FcεRI-Rezeptors oder der γ-Untereinheit davon durch Phosphorylierung der zytoplasmatischen Domäne der γ-Untereinheit aktiviert, wobei der Inhibitor ein Peptid ist, das die Sequenz Y-X2-L enthält, worin X2 irgendwelche zwei Aminosäuren oder eine Nachahmung davon darstellt, und wobei das Einbringen unter solchen Bedingungen bewirkt wird, daß die Signalweiterleitung gehemmt ist.

2. Verfahren nach Anspruch 1, wobei X2 die Aminosäuren von einer Y-X2-L-Sequenz der zytoplasmatischen Domäne der γ-Kette von FcεRI darstellt.

3. Pharmazeutische Zusammensetzung in der Form eines Aerosols zum Hemmen der Signalweiterleitung der γ-Untereinheit des Mastzellen-IgE-Rezeptors FcεRI, welche einen Träger und einen Inhibitor für eine Kinase enthält, wobei die Kinase endogen für die Zellen ist und die Signalweiterleitung des FcεRI-Rezeptors oder der γ-Untereinheit davon durch Phosphorylierung der zytoplasmatischen Domäne der γ-Untereinheit aktiviert und wobei der Inhibitor in dem Aerosol als ein Partikel vorliegt.

4. Zusammensetzung nach Anspruch 3, wobei das Partikel ein Liposom ist.

5. Zusammensetzung nach Anspruch 3 oder Anspruch 4, wobei der Inhibitor ein Peptid oder eine Nachahmung ist.

6. Zusammensetzung nach Anspruch 5, wobei das Peptid die Sequenz Y-X2-L enthält, worin X2 irgendwelche zwei Aminosäuren darstellt.

7. Zusammensetzung nach Anspruch 6, wobei X2 die Aminosäuren von einer Y-X2-L-Sequenz der zytoplasmatischen Domäne der γ-Kette von FcεRI darstellt.

8. Verwendung eines Inhibitors für eine Kinase, wobei die Kinase endogen für Mastzellen ist, die einen IgE-Rezeptor FcεRI tragen, und die Signalweiterleitung des FcεRI-Rezeptors oder der γ-Untereinheit davon durch Phosphorylierung der zytoplasmatischen Domäne der γ-Untereinheit aktiviert, zur Herstellung eines Medikamentes zur Behandlung einer immunologischen Erkrankung oder einer immunvermittelten Erkrankung, wobei der Inhibitor ein Peptid ist, das die Sequenz Y-X2-L enthält, worin X2 irgendwelche zwei Aminosäuren oder eine Nachahmung davon darstellt.

9. Verfahren nach Anspruch 8, wobei X2 die Aminosäuren von einer Y-X2-L-Sequenz der zytoplasmatischen Domäne der γ-Kette von FcεRI darstellt.

10. Inhibitor für eine Kinase, wobei die Kinase endogen für Mastzellen ist und die zytoplasmatische Domäne der γ-Untereinheit des Mastzellen-IgE-Rezeptors FcεRI phosphoryliert, wobei der Inhibitor (i) ein Peptid ist, das eine einzelne tyrosinhaltige Sequenz enthält, die zur Signalweiterleitung erforderlich ist, wobei die einzelne tyrosinhaltige Sequenz eine Y-X2-L-Sequenz ist, worin X2 irgendwelche zwei Aminosäuren oder Nachahmungen davon darstellt, und (ii) die Signalweiterleitung der γ-Untereinheit hemmt, wenn der Inhibitor in die Mastzellen eingebracht wird.

11. Inhibitor nach Anspruch 10, wobei X2 die Aminosäuren von einer Y-X2-L-Sequenz der zytoplasmatischen Domäne der γ-Kette von FcεRI darstellt.

## Revendications

1. Méthode *ex vivo* pour inhiber la transduction de signal de la sous-unité γ du récepteur de IgE de mastocytes FcεRI, comprenant l'introduction dans des mastocytes portant ledit récepteur d'un inhibiteur d'une kinase, kinase qui est endogène auxdites cellules et qui active ladite transduction de signal dudit récepteur FcεRI, ou de sa sous-unité γ, par phosphorylation du domaine cytoplasmique de ladite sous-unité γ, ledit inhibiteur étant un peptide comprenant la séquence Y-X2-L, dans laquelle X2 représente deux quelconques amino-acides, ou un de ses agents mimétiques, et ladite introduction étant effectuée dans des conditions telles que ladite transduction de signal soit inhibée.

2. Procédé suivant la revendication 1, dans lequel X2 représente les amino-acides d'une séquence Y-X2-L du domaine cytoplasmique de la chaîne γ de FcεRI.

3. Composition pharmaceutique, sous forme d'aérosol, pour inhiber la transduction de signal de la sous-unité γ du récepteur de IgE de mastocytes FcεRI, comprenant un support et un inhibiteur d'une kinase, kinase qui est endogène auxdites cellules et qui active ladite transduction de signal dudit récepteur FcεRI, ou de sa sous-unité γ, par phosphorylation du domaine cytoplasmique de ladite sous-unité γ, dans laquelle l'inhibiteur est présent dans l'aérosol sous forme d'une particule.

4. Composition suivant la revendication 3, dans laquelle la particule est un liposome.

5. Composition suivant la revendication 3 ou la revendication 4, dans laquelle ledit inhibiteur est un peptide ou agent mimétique.

6. Composition suivant la revendication 5, dans laquelle ledit peptide comprend la séquence Y-X2-L, dans laquelle X2 représente deux quelconques amino-acides.

7. Composition suivant la revendication 6, dans laquelle X2 représente les amino-acides d'une séquence Y-X2-L du domaine cytoplasmique de la chaîne γ de FcεRI.

8. Utilisation d'un inhibiteur d'une kinase, kinase qui est endogène aux mastocytes portant un récepteur de IgE FcεRI et qui active la transduction de signal dudit récepteur FcεRI, ou de sa sous-unité γ, par phosphorylation du domaine cytoplasmique de ladite sous-unité γ, pour la préparation d'un médicament destiné au traitement d'une maladie immunologique ou d'une maladie à médiation immunitaire, dans laquelle ledit inhibiteur est un peptide comprenant la séquence Y-X2-L, dans laquelle X2 représente deux quelconques amino-acides, ou un de ses agents mimétiques.

9. Méthode suivant la revendication 8, dans laquelle X2 représente les amino-acides d'une séquence Y-X2-L du domaine cytoplasmique de la chaîne γ de FcεRI.

10. Inhibiteur d'une kinase, kinase qui est endogène aux mastocytes et qui phosphoryle le domaine cytoplasmique de la sous-unité γ du récepteur de IgE de mastocytes FcεRI, ledit inhibiteur (i) étant un peptide comprenant une seule séquence contenant de la tyrosine requise par la transduction de signal, ladite séquence unique contenant de la tyrosine étant une séquence Y-X2-L, dans laquelle X2 représente deux quelconques amino-acides, ou un de ses agents mimétiques, et (ii) inhibant la transduction de signal de ladite sous-unité γ lorsque ledit inhibiteur est introduit dans lesdits mastocytes.

11. Inhibiteur suivant la revendication 10, dans lequel X2 représente les amino-acides d'une séquence Y-X2-L du domaine cytoplasmique de la chaîne γ de FcεRI.
